# FASCICULE DE BREVET EUROPEEN

(11) **EP 2 029 235 B1**
(45) Date de publication et mention de la délivrance du brevet: **15.04.2015**
(21) Numéro de dépôt: 07765945.6
(22) Date de dépôt: 23.05.2007
(51) Int. Cl.: A61Q 5/04, A61K 8/42, A61K 8/49

(54) **PROCEDE DE DEFRISAGE DES FIBRES KERATINIQUES AVEC UN MOYEN DE CHAUFFAGE ET UNE AMIDE**
VERFAHREN ZUR GLÄTTUNG VON KERATINFASERN MIT ERWÄRMUNGSMITTEL UND EINEM AMID
METHOD FOR STRAIGHTENING KERATINOUS FIBERS USING HEATING MEANS AND AN AMIDE

(30) Priorité: 24.05.2006 FR 0651913; 19.06.2006 US 814530 P
(43) Date de publication de la demande: 04.03.2009
(73) Titulaire: L'Oréal, 75008 Paris (FR)
(72) Inventeur: MALLE, Gérard, F-77580 Villiers S/Morin (FR); DUBLANCHET, Anne-Claude, F-92160 Antony (FR); BARBARAT, Philippe, F-92270 Bois-Colombes (FR)
(74) Mandataire: Bourdeau, Françoise
(86) Numéro de dépôt international: PCT/FR2007/000869
(87) Numéro de publication internationale: WO 2007/135296

(56) Documents cités:
- EP-A- 1 468 667
- EP-A- 1 570 834
- EP-A- 1 584 327
- EP-A1- 0 465 342
- DE-A1- 3 535 351
- US-A- 5 957 140
- DATABASE WPI Week 200346 Derwent Publications Ltd., London, GB; AN 2003-485967 XP002454409 & JP 2002 356408 A (MIRUBON KK) 13 décembre 2002 (2002-12-13)

## Description

L'invention a pour objet un procédé de défrisage des fibres kératiniques avec un moyen de chauffage et au moins une amide cyclique selon la revendication 1.

Le procédé de défrisage selon l'invention est réalisé sans utiliser d'agent réducteur, ni d'agent de lanthionisation. Il ne comprend aucune étape de réduction, ni de lanthionisation.

Par *"fibres kératiniques",* on entend, selon l'invention, des fibres d'origine humaine ou animale telles que les cheveux, les poils, les cils, la laine, l'angora, le cachemire ou la fourrure. Bien que l'invention ne soit pas limitée à des fibres kératiniques particulières, il sera néanmoins fait plus particulièrement référence aux cheveux.

Le terme *"défrisage"* englobe, selon l'invention, le défrisage, le lissage ou le décrêpage de cheveux caucasiens ou africains. Le terme *« défriser »* englobe, selon l'invention, défriser, lisser ou décrêper des cheveux caucasiens ou africains.

Par *« moyen de chauffage»,* on entend tout moyen permettant de chauffer les fibres kératiniques à une température d'au moins 110°C tels que les fers chauffants, par exemple les fers plats ou ronds, les générateurs de micro-ondes, les sources de rayonnement infra-rouge.

Deux techniques sont utilisées pour obtenir une déformation permanente des cheveux. Elles sont basées sur une rupture des liaisons covalentes disulfures présentes dans la kératine (cystine) :
- la première consiste, dans un premier temps à réaliser cette ouverture des liaisons disulfures à l'aide d'une composition contenant un agent réducteur, puis après avoir, de préférence, rincé les cheveux, à reconstituer dans un second temps les dites liaisons disulfures en appliquant sur les cheveux préalablement mis sous tension par des bigoudis ou autres ou mis en forme ou lissés par d'autres moyens, une composition oxydante encore appelée fixateur, de façon à donner à la chevelure la forme recherchée. Cette technique permet indifféremment de réaliser, soit l'ondulation des cheveux, soit leur défrisage ou leur décrêpage ou leur lissage.
- La seconde consiste à effectuer une opération dite de lanthionisation, à l'aide d'une composition contenant une base appartenant à la famille des hydroxydes. Elle conduit à remplacer des liaisons disulfures (-CH2-S-S-CH2-) par des liaisons lanthionines (-CH2-S-CH2-). Cette opération de lanthionisation fait intervenir deux réactions chimiques consécutives :
   ▪ La première réaction consiste en une béta-élimination sur la cystine provoquée par un ion hydroxyde, conduisant à la rupture de cette liaison et à la formation de déhydro-alanine. 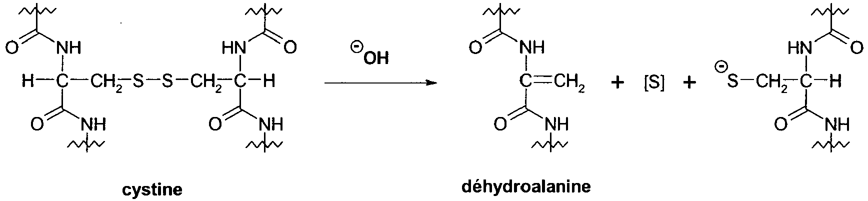
   ▪ La deuxième réaction est une réaction de la déhydro-alanine avec un groupe thiol. En effet, la double-liaison de la déhydro-alanine formée est une double-liaison réactive. Elle peut réagir avec le groupe thiol du résidu cystéine qui a été libéré pour former une nouvelle liaison appelée pont ou liaison ou résidu lanthionine.

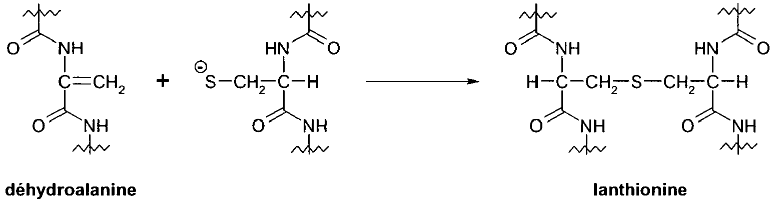

Par rapport à la première technique mettant en oeuvre un agent réducteur, cette technique de lanthionisation ne nécessite pas d'étape de fixation, puisque la formation des ponts lanthionine est irréversible. Elle s'effectue donc en une seule étape et permet indifféremment de réaliser soit l'ondulation des cheveux, soit leur défrisage ou leur décrêpage ou leur lissage. Cependant, elle est principalement utilisée pour le défrisage des cheveux naturellement crépus.

Pour la première technique, les compositions réductrices généralement utilisées pour la première étape d'une opération de permanente ou de défrisage contiennent à titre d'agent réducteur des thiols ou des sulfites ou des bisulfites. Ces agents sont généralement employés en milieu essentiellement aqueux à des concentrations comprises entre 0,5 et 1 M pour obtenir une bonne ouverture des liaisons disulfures. Parmi les thiols, ceux couramment utilisés sont l'acide thioglycolique, la cystéamine, le monothioglycolate de glycérol, l'acide thiolactique et la cystéine. L'acide thioglycolique est particulièrement efficace pour réduire les liaisons disulfures de la kératine à pH alcalin, notamment sous forme de thioglycolate d'ammonium, et constitue le produit le plus utilisé en permanente (" hair waving "). On a toutefois constaté que l'acide thioglycolique doit être utilisé en milieu suffisamment basique (en pratique à pH compris entre 8,5 et 9,5) si on veut obtenir une frisure satisfaisante en intensité. Outre l'inconvénient de dégager une odeur désagréable nécessitant l'usage de parfums plus ou moins efficaces pour masquer les odeurs, l'utilisation d'un thiol à pH alcalin conduit également à des dégradations de la fibre et tout particulièrement à l'altération des colorations artificielles.

Les sulfites ou bisulfites sont principalement utilisés pour le défrisage. Ils possèdent des désavantages similaires aux thiols avec une efficacité moindre.

Les thiols et les sulfites (ou bisulfites) présentent en outre l'inconvénient d'avoir une mauvaise stabilité en solution aqueuse.

D'une façon générale, la durabilité des effets de déformations obtenus avec les thiols et les sulfites par réduction des disulfures puis fixation est jugée très inférieure à celle pouvant être obtenue par la technique de lanthionisation.

Pour la deuxième technique, les compositions généralement utilisées pour effectuer la lanthionisation contiennent à titre de base un hydroxyde tel que l'hydroxyde de sodium, l'hydroxyde de guanidinium et l'hydroxyde de lithium. Ces actifs de lanthionisation, permettant d'ouvrir les liaisons disulfures par un mécanisme de béta-élimination, sont généralement employés en émulsion eau-huile à des concentrations comprises entre 0,4 et 0,6M, en les laissant agir généralement 10 à 15 minutes à température ambiante. L'hydroxyde de sodium reste l'agent le plus utilisé. L'hydroxyde de guanidinium est maintenant le composé préféré pour de nombreuses compositions. Ces deux hydroxydes, de sodium et de guanidinium, sont les deux agents principaux utilisés pour le défrisage ou le décrêpage des cheveux naturellement crépus. Ils possèdent plusieurs avantages par rapport au thioglycolate d'ammonium et aux sulfites, en particulier une absence d'odeur désagréable, le fait qu'une seule étape de mise en oeuvre est requise (durée du traitement plus courte), et une durabilité et une efficacité beaucoup plus importante de la déformation du cheveu.
Cependant, ces hydroxydes présentent l'inconvénient majeur d'être caustiques. Cette causticité affecte le cuir chevelu en provoquant des irritations parfois sévères. On peut y remédier partiellement par l'application préalable sur le scalp d'une crème protectrice grasse souvent appelée " base " ou " crème base ", le mot " base " ici utilisé n'ayant pas la signification d'agent basique au sens chimique. Lorsque la crème protectrice est associée à l'hydroxyde en une seule composition, celle-ci est généralement appelée " no-base ", par opposition à l'appellation ci-dessus. Cette technologie " no-base " est préférée.

La causticité des hydroxydes affecte également l'état du cheveu en le rendant d'une part rêche au toucher et d'autre part beaucoup plus fragile, cette fragilité pouvant aller jusqu'à l'effritement voire la rupture ou même la dissolution des cheveux si le traitement est prolongé. Les hydroxydes provoquent dans certains cas également des décolorations de la couleur naturelle du cheveu.

Les formules contenant de l'hydroxyde de sodium sont généralement dénommées en anglais " lye relaxers" et celles n'en contenant pas sont dénommées " no-lye relaxers ".

Les principales formules défrisantes dites " no-lye " mettent en oeuvre l'hydroxyde de guanidinium. L'hydroxyde de guanidinium étant instable, celui-ci est généré extemporanément par le mélange de carbonate de guanidine et d'une source d'hydroxyde très peu soluble telle que l'hydroxyde de calcium. La réaction entre ces deux composés conduit à la formation d'hydroxyde de guanidinium et de carbonate de calcium qui précipite dans la composition. La présence de ce précipité rend le rinçage final des cheveux beaucoup plus difficile et laisse sur les cheveux et le scalp des particules minérales qui lui donnent un toucher rêche et une apparence inesthétique ressemblant aux pellicules. Le succès récent de l'hydroxyde de guanidinium (" no-lye ") face à l'hydroxyde de sodium (" lye ") semble provenir d'une meilleure efficacité de défrisage et d'une meilleure tolérance cutanée. Cependant ces technologies mettant en oeuvre des bases de la famille des hydroxydes demeurent très agressives pour le cheveu et le cuir chevelu et demandent un contrôle très strict de la durée d'application pour éviter les irritations trop fortes et l'altération des cheveux pouvant aller jusqu'à la cassure. Cette agressivité provenant de la causticité des hydroxydes justifie que ces compositions pour la lanthionisation du cheveu ne soient pas utilisées pour la permanente (" hair waving ") mais réservées au défrisage (" hair straightening " ou " hair relaxing ").

De plus, les hydroxydes sont connus pour être de bons agents d'hydrolyse des fonctions amides (cf par exemple March's Advanced Organic Chemistry, 5ed., Wiley Interscience, New York, " Hydrolysis of Amides " pages 474 et suivantes) qui conduisent donc à la rupture des liaisons peptidiques par attaque nucléophile directe. Il est ainsi probable que les altérations constatées au niveau des cheveux et des matières kératiniques au sens large soient en grande partie dues à une hydrolyse partielle des liaisons amides de la kératine.

Il existe donc un réel besoin pour le défrisage de compositions nettement moins agressives pour le cheveu.

Diverses études ont été conduites en vue de remédier à la fois aux inconvénients des agents réducteurs (première technique) et/ou des hydroxydes (deuxième technique).

Ainsi pour remplacer l'acide thioglycolique, de nombreux agents réducteurs ont été proposés, mais l'acide thioglycolique sous sa forme de thioglycolate d'ammonium reste à la fois le composé de référence et le plus largement utilisé dans les formulations cosmétiques, aussi bien pour la mise en forme que pour le lissage.

Il a également été proposé dans de très nombreux brevets d'associer des agents réducteurs habituels (thiols ou sulfites ou bisulfites) avec de l'urée ou des alkylurées pour diminuer l'irritation et les dommages causés aux cheveux aussi bien pour la mise en forme que pour le défrisage. On citera par exemple :
- la demande CA 1315204 qui décrit une composition contenant du thioglycolate d'ammonium (5,5-11,5%) et de l'urée ou une monoalkylurée (1-3%) pour la mise en forme des cheveux,
- la demande US 3847165 qui décrit une composition contenant du thioglycolate d'ammonium (1,2-1,4M) et de l'urée (2,0-2,7M) pour la mise en forme des cheveux à un pH acide,
- la demande NL 6410355 qui décrit une composition contenant un sulfite (0,8-1,5M) et de l'urée (0,6-3,0M) pour la mise en forme et le défrisage des cheveux,
- la demande JP 2000/229819 qui décrit une composition contenant un sulfite ou bisulfite (0,5-15%), de l'urée (0,5-15%) et un alcool (éthanol et/ou isopropanol, 1-30%) pour la mise en forme et le défrisage des cheveux,

Il a également été proposé dans de très nombreux brevets d'associer des hydroxydes, servant d'actif de lanthionisation, avec certains additifs servant généralement à protéger les cheveux. On citera, à titre d'exemple:
- la demande WO2002/003937, qui décrit une composition contenant des monosaccharides en C3-C5,
- la demande WO2001/064171, qui décrit une composition contenant des agents complexants,
- le brevet US5641477, qui décrit une composition contenant un hydrolysat d'amidon hydrogéné,
- la demande WO02085317, qui décrit une composition contenant des nucléophiles organiques qui réagissent lors de la deuxième étape avec la déhydro-alanine formée avec des hydroxydes, pour conduire à de nouveaux pontages.

Si toutes ces propositions conduisent à des améliorations plus ou moins marquées, elles ne permettent pas de diminuer de façon suffisante les dégâts liés à la causticité même des hydroxydes.

Comme indiqué précédemment, l'utilisation d'agents réducteurs conduit à une durabilité médiocre pour le défrisage ou le décrêpage et l'emploi d'hydroxydes, de part leur causticité, limite leur utilisation au domaine du défrisage.

EP-A-1 468 667 décrit un procédé de traitement des cheveux avec une céramide et un chauffage.

EP-A-0465342 décrit un procédé de traitement des cheveux à l'aide d'une succinimide, sans chauffage. EP-A-1 584 327 décrit un procédé à l'aide d'une niacinamide et d'un chauffage DE-A-35 35 351 concerne un procédé capillaire utilisant des carboxydithiols; JP 2002 35 64 08 décrit la mise en oeuvre d'un réducteur et d'un chauffage pour la mise en oeuvre des cheveux.

Après d'importantes études, on vient maintenant de découvrir, de façon tout à fait surprenante et inattendue, que l'on pouvait défriser durablement le cheveu en associant l'action d'une amide cyclique et d'un moyen de chauffage à une température supérieure à 110°C. On obtient ainsi d'excellents résultats en terme de défrisage, de propriétés cosmétiques des cheveux et d'intégrité de la fibre.

Sans être liée par la théorie, la Demanderesse pense qu'il existe une action conjointe, sur les fibres kératiniques, d'une amide cyclique et d'un moyen de chauffage, qui permet de les défriser de façon efficace et durable.

La Demanderesse a trouvé qu'il était possible de remédier aux inconvénients de l'art antérieur et de remplir les objectifs précités en mettant en oeuvre un procédé de défrisage des fibres kératiniques comprenant :
une étape d'application sur les fibres kératiniques d'une composition de défrisage contenant au moins une amide cyclique particulière, le pH de cette composition étant inférieur ou égal à 9,
une étape d'élévation de la température des fibres kératiniques, à l'aide d'un moyen de chauffage, à une température comprise entre 110 et 250°C.

Ainsi, l'invention a pour objet un procédé de défrisage des fibres kératiniques comprenant :
(i) une étape d'application sur les fibres kératiniques d'une composition de défrisage contenant au moins une amide cyclique, le pH de cette composition étant inférieur ou égal à 9,
(ii) une étape d'élévation de la température des fibres kératiniques, à l'aide d'un moyen de chauffage, à une température comprise entre 110 et 250°C,
caractérisé par le fait que l'amide cyclique est choisie parmi
La 2-PYRROLIDONE
L'ACIDE PYROGLUTAMIQUE
La 3-METHYL-2-PYRROLIDONE
La 5-METHYL-2-PYRROLIDONE
La 5-(HYDROXYMETHYL)-2-PYRROLIDINONE
L'ACIDE 5-OXO-PYRROLIDINE-3-CARBOXYLIQUE
La 5-AMINOMETHYL-PYRROLIDIN-2-ONE
La 4-HYDROXY-2-PYRROLIDINONE

Et leurs stéréoisomères et sels organiques ou minéraux et/ou solvates.
la composition renfermant l'amide cyclique comme seul actif de défrisage et l'amide étant présent en une concentration molaire comprise entre 2 et 8 M.

Avantageusement, on élève la température à l'aide du moyen de chauffage à une température comprise entre 120°C et 220°C, plus avantageusement entre 140°C et 220°C.

De préférence, ladite composition est appliquée sur des fibres kératiniques humides.

On peut également avantageusement intercaler entre l'étape d'application de la composition et l'étape d'élévation de température, une étape destinée à éliminer l'excédant de la composition, par exemple au moyen d'une serviette.

### Concentrations d'utilisation

La concentration molaire d'utilisation est avantageusement comprise entre 4 et 8M.

De préférence, le pH d'utilisation est inférieur ou égal à 7.

Les compositions selon l'invention se présentent soit sous la forme d'une solution aqueuse, soit sous forme d'une crème épaissie de façon à maintenir les cheveux aussi raides que possible. On réalise ces crèmes, sous forme d'émulsions « lourdes ».

Les compositions de l'invention renferment ladite amide cyclique comme seul actif de défrisage.

Dans le but d'améliorer les propriétés cosmétiques des fibres kératiniques ou encore d'atténuer ou d'éviter leur dégradation, la composition utilisée selon l'invention peut également comprendre un ou plusieurs actifs cosmétiques supplémentaires.

Généralement, le ou lesdits actifs cosmétiques supplémentaires représentent de 0,01 à 30%, de préférence de 0,1 à 10%, en poids du poids total de la composition cosmétique.

Généralement, la composition appliquée sur les fibres kératiniques est appliquée à hauteur de 0,05 à 20 g, de préférence de 0,1 à 10 g de composition par gramme de fibre kératinique sèche.

Après application de la composition, et avant l'élévation de la température des fibres kératiniques au moyen d'un moyen de chauffage, on peut laisser poser ladite composition, généralement pendant 30 secondes à 60 minutes, de préférence 5 à 45 minutes.

Le procédé selon l'invention comprend, après l'étape d'application de la composition, une étape d'élévation de la température des fibres kératiniques, au moyen d'un moyen de chauffage, à une température comprise entre 110°C et 250°C.

Avantageusement, on utilise un fer comme moyen de chauffage.

Au sens de la présente invention, on entend par « fer » un dispositif de chauffage des fibres kératiniques mettant en contact lesdites fibres et le dispositif de chauffage.

L'extrémité du fer venant en contact avec les cheveux présente généralement deux surfaces planes. Ces deux surfaces planes peuvent être métalliques. Elles peuvent être lisses ou crantées.

A titre d'exemple de fers utilisables dans le procédé selon l'invention, on peut citer tous types de fer plats et, en particulier, de manière non limitative, ceux décrits dans les brevets US 5 957 140, et US 5 046 516.

L'application du fer peut être effectuée par touches séparées successives de quelques secondes, ou par déplacement ou glissement progressif le long des mèches.

De préférence, l'application du fer dans le procédé selon l'invention se fait en mouvement continu de la racine à la pointe, en un ou plusieurs passages.

Le procédé selon l'invention peut également comprendre une étape supplémentaire de pré-séchage partiel des fibres kératiniques avant l'étape d'élévation de la température, de manière à éviter d'importants dégagements de vapeurs qui pourraient brûler les mains du coiffeur et le cuir chevelu de la personne. Cette étape de pré-séchage peut se faire par exemple au moyen d'un séchoir, d'un casque, ou bien encore par séchage libre.

L'invention concerne également un kit comprenant au moins :
- un moyen de chauffage procurant une température comprise entre 110 et 250°C,
- une composition de défrisage contenant au moins une amide cyclique, le pH de cette composition étant inférieur ou égal à 9, caractérisé par le fait que l'amide cyclique est choisie parmi
   La 2-PYRROLIDONE
   L'ACIDE PYROGLUTAMIQUE
   La 3-METHYL-2-PYRROLIDONE
   La 5-METHYL-2-PYRROLIDONE
   La 5-(HYDROXYMETHYL)-2-PYRROLIDINONE
   L'ACIDE 5-OXO-PYRROLIDINE-3-CARBOXYLIQUE
   La 5-AMINOMETHYL-PYRROLIDIN-2-ONE
   La 4-HYDROXY-2-PYRROLIDINONE

Et leurs stéréoisomères et sels organiques ou minéraux et/ou solvates.
la composition renfermant l'amide cyclique comme seul actif de défrisage et l'amide étant présent en une concentration molaire comprise entre 2 et 8 M.

L'invention pourra être mieux comprise à l'aide des exemples non limitatifs qui suivent et qui constituent des modes de réalisation préférentiels des compositions selon l'invention.

### EXEMPLE 1:

On réalise une composition de défrisage simplifiée contenant la 2-pyrrolidone, à une concentration de 8M dans l'eau, en tant qu'actif de défrisage. On applique cette composition sur des cheveux africains naturellement frisés pendant 15 minutes à une température de 40°C puis on essuie rapidement les cheveux avec une serviette.

On procède ensuite à un lissage mèche à mèche de la chevelure à l'aide d'un fer plat chauffé à 180°C pendant 10 à 15 secondes. Les cheveux sont défrisés efficacement et doux au toucher.

### EXEMPLE 2:

On réalise une composition de défrisage simplifiée contenant la 2-pyrrolidone, à une concentration de 6M dans l'eau, en tant qu'actif de défrisage. On applique cette composition sur des cheveux africains naturellement frisés pendant 25 minutes à une température de 40°C puis on essuie rapidement les cheveux avec une serviette.

On procède ensuite à un lissage mèche à mèche de la chevelure à l'aide d'un fer plat chauffé à 180°C pendant 10 à 15 secondes. Les cheveux sont défrisés efficacement et doux au toucher.

## Revendications

1. Procédé de défrisage des fibres kératiniques comprenant :
(i) une étape d'application sur les fibres kératiniques d'une composition de défrisage contenant au moins une amide cyclique, le pH de cette composition étant inférieur ou égal à 9,
(ii) une étape d'élévation de la température des fibres kératiniques, à l'aide d'un moyen de chauffage, à une température comprise entre 110 et 250°C,
**caractérisé par le fait que** les amides cycliques sont choisies parmi :
La 2-PYRROLIDONE
L'ACIDE PYROGLUTAMIQUE
La 3-METHYL-2-PYRROLIDONE
La 5-METHYL-2-PYRROLIDONE
La 5-(HYDROXYMETHYL)-2-PYRROLIDINONE
L'ACIDE 5-OXO-PYRROLIDINE-3-CARBOXYLIQUE
La 5-AMINOMETHYL-PYRROLIDIN-2-ONE
La 4-HYDROXY-2-PYRROLIDINONE
ainsi que leurs stéréoisomères et sels organiques ou minéraux et solvates,
la composition renfermant l'amide cyclique comme seul actif de défrisage et l'amide étant présent en une concentration molaire comprise entre 2 et 8 M.

2. Procédé selon la revendication 1, **caractérisé par le fait que** l'amide cyclique est la 2-PYRROLIDONE.

3. Procédé selon la revendication 1 ou 2, **caractérisé par le fait qu'**on élève la température à l'aide du moyen de chauffage à une température comprise entre 120°C et 220°C, plus avantageusement entre 140°C et 220°C.

4. Procédé selon l'une quelconque des revendications précédentes, **caractérisé par le fait que** la composition est appliquée sur des fibres kératiniques humides.

5. Procédé selon l'une quelconque des revendications précédentes, **caractérisé par le fait que** les fibres sont partiellement préséchées avant l'étape d'élévation de la température.

6. Procédé selon l'une quelconque des revendications précédentes, **caractérisé par le fait que** la concentration molaire de l'amide est comprise entre 4 et 8 M.

7. Procédé selon l'une quelconque des revendications précédentes, **caractérisé par le fait que** le pH est inférieur ou égal à 7.

8. Kit comprenant au moins :
- un moyen de chauffage procurant une température comprise entre 110 et 250°C.
- une composition de défrisage contenant au moins une amide cyclique choisie parmi :
La 2-PYRROLIDONE
L'ACIDE PYROGLUTAMIQUE
La 3-METHYL-2-PYRROLIDONE
La 5-METHYL-2-PYRROLIDONE
La 5-(HYDROXYMETHYL)-2-PYRROLIDINONE
L'ACIDE 5-OXO-PYRROLIDINE-3-CARBOXYLIQUE
La 5-AMINOMETHYL-PYRROLIDIN-2-ONE
La 4-HYDROXY-2-PYRROLIDINONE
ainsi que leurs stéréoisomères et sels organiques ou minéraux et solvates,
le pH de cette composition étant inférieur ou égal à 9,
la composition renfermant l'amide cyclique comme seul actif de défrisage et l'amide étant présent en une concentration molaire comprise entre 2 et 8

9. Kit selon la revendication précédente, **caractérisée par le fait que** l'amide cyclique est la 2-PYRROLIDONE.

## Patentansprüche

1. Verfahren zur Entkräuselung von Keratinfasern, umfassend:
(i) einen Schritt des Aufbringens einer Entkräuselungszusammensetzung, die mindestens ein cyclisches Amid enthält, auf die Keratinfasern, wobei der pH-Wert dieser Zusammensetzung kleiner gleich 9 ist,
(ii) einen Schritt des Erhöhens der Temperatur der Keratinfasern mit Hilfe eines Erhitzungsmittels auf eine Temperatur zwischen 110 und 250°C, **dadurch gekennzeichnet, dass** die cyclischen Amide aus:
2-PYRROLIDON
PYROGLUTAMINSÄURE
3-METHYL-2-PYRROLIDON
5-METHYL-2-PYRROLIDON
5-(HYDROXYMETHYL)-2-PYRROLIDINON
5-OXOPYRROLIDIN-3-CARBONSÄURE
5-AMINOMETHYLPYRROLIDIN-2-ON
4-HYDROXY-2-PYRROLIDINON
sowie Stereoisomeren und organischen oder
anorganischen Salzen und Solvaten davon ausgewählt werden,
wobei die Zusammensetzung das cyclische Amid als einziges Entkräuselungsmittel enthält und das Amid in einer molaren Konzentration zwischen 2 und 8 M vorliegt.

2. Verfahren nach Anspruch 1, **dadurch gekennzeichnet, dass** es sich bei dem cyclischen Amid um 2-PYRROLIDON handelt.

3. Verfahren nach Anspruch 1 oder 2, **dadurch gekennzeichnet, dass** man die Temperatur mit Hilfe des Erhitzungsmittels auf eine Temperatur zwischen 120°C und 220°C und noch vorteilhafter zwischen 140°C und 220°C erhöht.

4. Verfahren nach einem der vorhergehenden Ansprüche, **dadurch gekennzeichnet, dass** die Zusammensetzung auf feuchte Keratinfasern aufgebracht wird.

5. Verfahren nach einem der vorhergehenden Ansprüche, **dadurch gekennzeichnet, dass** die Fasern vor dem Schritt des Erhöhens der Temperatur teilweise vorgetrocknet werden.

6. Verfahren nach einem der vorhergehenden Ansprüche, **dadurch gekennzeichnet, dass** die molare Konzentration des Amids zwischen 4 und 8 M liegt.

7. Verfahren nach einem der vorhergehenden Ansprüche, **dadurch gekennzeichnet, dass** der pH-Wert kleiner gleich 7 ist.

8. Kit, umfassend mindestens:
- ein Erhitzungsmittel, das eine Temperatur zwischen 110 und 250°C liefert,
- eine Entkräuselungszusammensetzung, die mindestens ein cyclisches Amid enthält, das aus:
2-PYRROLIDON
PYROGLUTAMINSÄURE
3-METHYL-2-PYRROLIDON
5-METHYL-2-PYRROLIDON
5-(HYDROXYMETHYL)-2-PYRROLIDINON
5-OXOPYRROLIDIN-3-CARBONSÄURE
5-AMINOMETHYLPYRROLIDIN-2-ON
4-HYDROXY-2-PYRROLIDINON
sowie Stereoisomeren und organischen oder
anorganischen Salzen und Solvaten davon ausgewählt ist,
wobei der pH-Wert dieser Zusammensetzung kleiner gleich 9 ist,
wobei die Zusammensetzung das cyclische Amid als einziges Entkräuselungsmittel enthält und das Amid in einer molaren Konzentration zwischen 2 und 8 M vorliegt.

9. Kit nach dem vorhergehenden Anspruch, **dadurch gekennzeichnet, dass** es sich bei dem cyclischen Amid um 2-PYRROLIDON handelt.

## Claims

1. Process for relaxing keratin fibres, comprising:
(i) a step of applying to the keratin fibres a hair-relaxing composition containing at least one cyclic amide, the pH of this composition being less than or equal to 9,
(ii) a step of raising the temperature of the keratin fibres, using a heating means, to a temperature of between 110 and 250°C, **characterized in that** the cyclic amides are chosen
from:
2-pyrrolidone
pyroglutamic acid
3-methyl-2-pyrrolidone
5-methyl-2-pyrrolidone
5-(hydroxymethyl)-2-pyrrolidinone
5-oxopyrrolidine-3-carboxylic acid 5-aminomethylpyrrolidin-2-one
4-hydroxy-2-pyrrolidinone
and the stereoisomers, organic or mineral salts
and solvates thereof,
the composition containing the cyclic amide as the only hair-relaxing active ingredient and the amide being present in a molar concentration of between 2 and 8 M.

2. Process according to Claim 1, **characterized in that** the cyclic amide is 2-pyrrolidone.

3. Process according to Claim 1 or 2, **characterized in that** the temperature is raised using a heating means to a temperature of between 120°C and 220°C and more advantageously between 140°C and 220°C.

4. Process according to any one of the preceding claims, **characterized in that** the composition is applied to wet keratin fibres.

5. Process according to any one of the preceding claims, **characterized in that** the fibres are partially predried before the step of raising the temperature.

6. Process according to any one of the preceding claims, **characterized in that** the molar concentration is between 4 and 8 M.

7. Process according to any one of the preceding claims, **characterized in that** the pH is less than or equal to 7.

8. Kit comprising at least:
- one heating means that affords a temperature of between 110 and 250°C,
- one hair-relaxing composition containing at least one cyclic amide chosen from:
2-pyrrolidone
pyroglutamic acid
3-methyl-2-pyrrolidone
5-methyl-2-pyrrolidone
5-(hydroxymethyl)-2-pyrrolidinone
5-oxopyrrolidine-3-carboxylic acid
5-aminomethylpyrrolidin-2-one
4-hydroxy-2-pyrrolidinone
and the stereoisomers, organic or mineral salts and solvates thereof,
the pH of this composition being less than or equal to 9,
the composition containing the cyclic amide as the only hair-relaxing active ingredient and the amide being present in a molar concentration of between 2 and 8 M.

9. Kit according to the preceding claim, **characterized in that** the cyclic amide is 2-pyrrolidone.
